# EUROPEAN PATENT APPLICATION

(11) **EP 3 072 440 A1**
(43) Date of publication of application: **28.09.2016**
(21) Application number: 15382142.6
(22) Date of filing: 24.03.2015
(51) Int. Cl.: A61B 5/00, A63B 69/00, A63B 69/18, A61B 5/11, A63B 24/00

(54) **METHOD AND PORTABLE DEVICE FOR TRACKING SPORTS ACTIVITY**

(71) Applicant: Gradient Technologies S.L., 46002 Valencia (ES)
(72) Inventor: Alonso Berrotarán, Mikel, 46002 Valencia (ES); Del Rio Rodriguez, Daniel, 46002 Valencia (ES); Del Cura González, Rubén, 46002 Valencia (ES)
(74) Representative: Balder IP Law, S.L.

(57) **Abstract**

A portable device for tracking sports activity performed by a user to whom said portable device is associated, comprising a microprocessor (11), memory means (15) for storing information, a GPS module (13) for calculating the position of the device and a display (12) for providing visual information. The portable device is configured to establish, from the information captured at least by said GPS module (13), at least one marker associated to the sports activity performed by said user and to store said at least one marker in said memory means (15). The portable device being characterized in that it comprises an RF module (14) implementing a short-distance wireless protocol, said RF module (14) being configured to be set to work in broadcast mode, thus enabling the portable device to broadcast a message comprising an identifier of the portable device and said at least one marker associated to the sports activity performed by said user. Method for tracking sports activity.

## Description

### TECHNICAL FIELD

The present invention relates to the field of tracking devices and methods and, in particular, to methods and portable devices for tracking sports activity or performance.

### STATE OF THE ART

Sportsmen and sportswomen, such as runners, usually wear devices that keep them informed about weather conditions and other parameters. These devices usually track different biological inputs of the user, such as pulsation or energy consumption.

For nautical sports, such as surfing, water skiing or sailing, users are often interested in getting additional inputs, such as tide information. An example of a device that provides a tide display is disclosed in United States patent application US2007/0237033A1. This device comprises a storage mechanism that stores tide information by surf spot. The device has a microprocessor that is programmed to display the tide height both graphically and numerically. The tide information is downloaded from a web server, for example to a PDA or cellular phone, from which it is loaded into the memory device by via a wired or wireless protocol.

The performance of sportsmen, especially for action sports, is usually tracked and provided to a remote system for long-term data storage, processing and presentation. For example, international patent application WO2014/137387A1 discloses a method for simultaneously visually displaying the performance of one or more athletic maneuver during different time periods, by means of a computing system which communicates with several sensor devices via a network. Thanks to the information sent from the sensor devices to the computing system, multiple video segments of an actor or actors performing athletic maneuvers are synchronized. Also, United States patent application US2014/0120838A1 discloses a system to simultaneously measure sports motion performed by several users. The system is based on a three-stage hierarchy of devices. Each user wears at least one device (stage-one device) that uses sensors to collect initial data and transfer these data via a short range RF transceiver to a stage-two device also carried by the user. A third device (stage-three device) includes a server computer that communicates with several stage-two devices (that is to say, with several users). This third device is configured to collect data from the stage-two devices, perform data processing of the data of each individual user and store computed performance characteristics parameters of each user.

However, some sportsmen and sportswomen, such as surfers and skiers, may need or want to automatically track and process their performance, for example to share it on the spot with other surfers/skiers just for fun or during a contest to compete with them.

### DESCRIPTION OF THE INVENTION

It is therefore an object of the invention to provide a portable device and a method for tracking the performance of sportsmen and sportswomen, in order to for example share it with users of similar devices.

According to an aspect of the present invention, there is provided a portable device for tracking sports activity performed by a user to whom said portable device is associated. The portable device comprises a microprocessor, memory means for storing information, a GPS module for calculating the position of the device and a display for providing visual information. The portable device is configured to establish, from the information captured at least by the GPS module, at least one marker associated to the sports activity performed by the user and to store the at least one marker in the memory means. The portable device comprises an RF module implementing a short-distance wireless protocol. The RF module is configured to be set to work in broadcast mode, thus enabling the portable device to broadcast a message comprising an identifier of the portable device and the at least one marker associated to the sports activity performed by the user.

In a particular embodiment, the message also comprises the maximum number of times the message is to be retransmitted.

In a particular embodiment, the RF module is configured to be switched from broadcast mode to master-slave mode in order to either download information to the memory means or to upload the at least one marker associated to the sports activity performed to another device.

The device preferably comprises at least one accelerometer configured to provide additional information for the device to establish the at least one marker.

The memory means is preferably configured to store at least one of the following: a plurality of sports spots, a weather forecast, tide information and information about past sports sessions.

In a particular embodiment, the device comprises a GSM/3G module.

The device is preferably implemented as a wearable device, such as a wrist watch, a bracelet or wrist device, an ankle brace or a necklace.

According to another aspect of the present invention, a system comprising a plurality of portable devices like the ones previously described, is provided. The RF module of each portable device of that plurality of portable devices is set to work in broadcast mode, in such a way that when one of the portable devices broadcasts a message comprising at least one marker associated to the sports activity performed by its user, that message can be received by the portable devices which are within the coverage area of the device broadcasting the message, in such a way that the tracked activity of the users of those devices is shared on the spot by the corresponding users.

According to another aspect of the present invention, a method for tracking sports activity performed by a user having an associated portable device, is provided. The method is performed in the portable device associated to a user performing sports activity. The method comprises the following steps: calculating at least one marker associated to the sports activity performed from the information captured at least by the GPS module (13); storing the at least one marker in a memory means (15) of the device; broadcasting a message comprising an identifier of the portable device and said at least one marker associated to the sports activity performed, wherein the message is able to be received by any other portable device set in broadcasting mode and located within the coverage area of the portable device broadcasting said message.

In a preferred embodiment, the method further comprises the steps of, when a broadcasted message is received by another device: storing in said another device said at least one marker associated to the sports activity performed; and broadcasting said message from said another device; in such a way that a plurality of devices associated to respective users can update their portable devices with the tracked activity of other devices.

In a particular embodiment, in which the at least one marker associated to the sports activity performed is a number of surfed waves, the step of calculating said at least one marker associated to the sports activity performed is done as follows: obtaining by said GPS module (13) the position of the device and time; calculating the instant velocity and distance performed by the user from different obtained values of position of the device and time; comparing the instant velocity with a velocity threshold and, if said instant velocity is above said threshold, establishing that the user of the device is either surfing or is being dragged by the wave; measuring the amount of time the instant velocity is above said threshold or the distance done at a velocity higher than said velocity threshold and, if said amount of time is larger than a time threshold or said distance done is larger than a distance threshold, establishing that the user has surfed a wave.

The at least one marker associated to the sports activity performed is a number of surfed waves, a number of ski descents, a duration of an activity, a trajectory or distance of an activity, a value of velocity, a value of height, a starting time, an ending time, a duration of a pause, a score or a ranking.

In a preferred embodiment, the sports activity is surfing and the at least one marker associated to the surfing activity is the number of surfed waves.

In an alternative embodiment, the sports activity is skiing or snow-boarding and the at least one marker associated to the sports activity is the number of descents performed. In a particular embodiment, the message further comprises a table comprising a ranking of several user with their scores associated to a sports activity.

Additional advantages and features of the invention will become apparent from the detail description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows an exemplary tracking device according to the present invention.
Figure 2 shows a diagram of the blocks forming the tracking device.
Figure 3 illustrates how the information tracked by a device of the invention is shared with other similar devices during a sports competition.
Figure 4 shows an exemplary implementation of the menu displayed on the display of the device according to the invention.
Figure 5 shows examples of the Session information as shown on a device display according to an embodiment of the invention.
Figure 6 shows some examples of the history information as can be shown on a device display according to an embodiment of the invention.
Figure 7 shows some examples of the forecast information as can be displayed on a device display device according to an embodiment of the invention.
Figure 8 shows the statistics option displayed on the display of the device according to an embodiment of the invention.
Figure 9 shows an exemplary design of the watch function (time displayed) of the device of the invention.

### DESCRIPTION OF A WAY OF CARRYING OUT THE INVENTION

In this text, the term "comprises" and its derivations (such as "comprising", etc.) should not be understood in an excluding sense, that is, these terms should not be interpreted as excluding the possibility that what is described and defined may include further elements, steps, etc.

In the context of the present invention, the term "approximately" and terms of its family (such as "approximate", etc.) should be understood as indicating values very near to those which accompany the aforementioned term. That is to say, a deviation within reasonable limits from an exact value should be accepted, because a skilled person in the art will understand that such a deviation from the values indicated is inevitable due to measurement inaccuracies, etc. The same applies to the terms "about" and "around" and "substantially".

The following description is not to be taken in a limiting sense but is given solely for the purpose of describing the broad principles of the invention. Next embodiments of the invention will be described by way of example, with reference to the above-mentioned drawings showing apparatuses and results according to the invention.

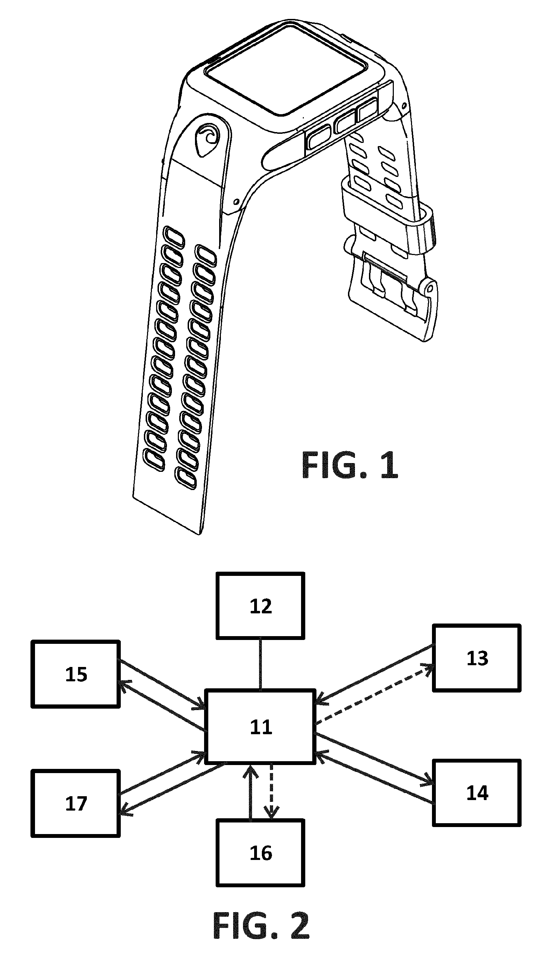

Non-limiting examples of sports to which the present invention refers, are: skiing, snowboarding, skateboarding, surfing or surfboarding, windsurfing, kitesurfing or skydiving. The device for tracking sports performance or activity is a portable device preferably implemented as a wearable device. Non-limiting examples of such wearable devices are a watch or smart watch, a bracelet or wrist device, an ankle brace or a necklace. Figure 1 shows a design of an exemplary tracking device according to the present invention. Alternatively, the tracking device can be configured to be attached to an object, such as to the leash of a surf table or directly to the surf table or to a snowboard. Alternatively, the tracking device can be a standalone device, such as a PDA, cellular phone or any portable device comprising a microprocessor. The device preferably comprises an attachment means or mechanism, such as a removable wrist band or ankle band, a VELCRO™ strip, reusable adhesive, one or more clips, or any other conventional attaching means.

Figure 2 shows a diagram of the blocks forming the tracking device. The device comprises a microprocessor 11 for controlling other peripherals. It is a conventional microprocessor 11 having memory means for storing the algorithms for making such control and in general for performing the tracking method. The device also comprises: a display 12, for providing the user with visual information; memory means 15, such as a flash memory; a GPS module 13; an RF module 14; at least one sensor 15; and optionally, a GSM/3G module 17. All these elements/modules are controlled by the microprocessor 11, as indicated by the arrows: arrows starting at an element (i.e. the microprocessor 11) refer to requests made by that element, while arrows arriving at an element refer to information received by that element.

The sensor 15 comprises an accelerometer and a gyroscope. It is a conventional one. It is preferably a triaxial one. It is for example an MPU-6500™ Motion Tracking. In the particular embodiment in which the device is being used by a surfer, the accelerometer 15 detects the swimming stroke when the surfer is swimming or moving towards the position at which he/she is going to wait for the wave. This information is used by the device for interpreting that the surfer is getting ready to surf and therefore for counting the amount or surfed waves. In the particular embodiment in which the device is being used by a skier, the accelerometer detects the arm movement of the skier. From this information, the device can additionally estimate the energy consumption made by the user of the device.

The GPS module 13 is a conventional one. It comprises at least one GPS antenna and the conventional electronics, such as transmitting means and receiving means, for identifying the coordinates of its position and the exact time.

The RF module 14 is preferably a communications module working under a short-distance wireless protocol, such as the ones referred to as Bluetooth or Bluetooth Low Energy (BLE), version 4.0 or 4.1 or any other one. It therefore comprises the conventional elements which form part of RF communications modules, such as receiving/transmitting antenna(s) and receiving/transmitting means, among others. It also comprises memory means (such as an internal memory) and processing means (such as a processor or microprocessor). This RF module 14 is configured to provide a conventional standardized short-distance wireless communication protocol with any device having a similar RF module (for example, with a PDA, laptop, mobile phone or another tracking device). The RF module 14 additionally implements and provides a proprietary protocol which enables the device to communicate with other tracking devices having an RF module in turn having the same proprietary protocol. The processor or microprocessor of the RF module 14 permits to work with the conventional standardized short-distance wireless communication protocol or with the proprietary protocol. The software associated to these protocols is stored in the memory means of the RF module 14. This provides with several advantages over conventional Bluetooth protocols (or similar), as will be described next. In a preferred embodiment, the RF module 14 cannot work simultaneously in the standardized mode and in the proprietary mode. It must, on the contrary, switch from one mode to another one, as explained soon. This switching ability is controlled by the processing means. This switching from one protocol to another one can be automatically or manually. As explained next, the standardized short-distance wireless communication protocol is for example used to exchange information with an application, a server, a mobile phone or a computer, while the proprietary protocol is preferably used for the exchange of gamification information among several devices on the spot (that is to say, preferably during their sports session).

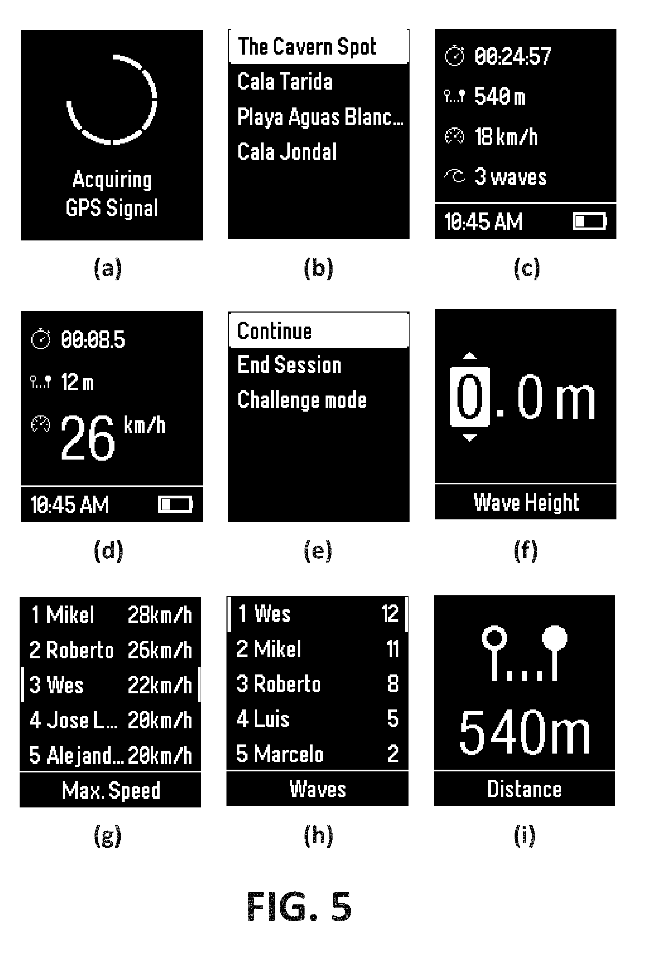

In the memory means 15 a local database is stored. This database keeps all the information directly or indirectly related to the sports activity. For example, it stores potential sports locations and their weather forecasts, history of sports sessions performed by the users, statistics of their performance and scores, and so on. In a particular embodiment, the data base keeps a plurality of spots (locations suitable for performing sports activities). If the user is a surfer, the spots are usually locations at different beaches, typically well-known for their appropriate surfing conditions. If the user is a skier or snow-boarder, the spots can be locations at different ski resorts typically well-known for their appropriate skiing/snow-boarding conditions. In a possible implementation, depending on the location of the user (detected by the GPS module 13), the local database in the memory 15 is updated with the closest spots. Or alternatively the data base can keep the spots at which de user has been doing sports recently. In other words, the local database can be updated following different criteria. This update is done either from the internet (in the optional case in which the device has a GSM/3G module 17) or from a mobile application (APP), in which case the update is done from an external device (mobile phone, PDA, laptop...) carrying said application (APP) to the device through its RF module 14 working in conventional standardized short-distance wireless communication protocol mode (such as Bluetooth Low Energy (BLE)). The local database in the memory 15 is also updated with meteorological information of different sports, such as wind conditions, tides and water temperature (if the user is going to surf) or atmosphere temperature and snow conditions (quantity and quality) (if the user is going to practice winter sports), and any other parameter that might be interesting depending on the activity the user is going to perform. All this information is downloaded in a similar way as the spots. The downloaded information can be provided to the device display 12, graphically and/or numerically. The user can select the information to be displayed by handling a menu managed through buttons or keys, as will be shown with reference to figures 5-9.

Next, it is explained how the device of the invention works. When a person having or wearing a device like the one represented in figure 2, arrives at a place in which the person is going to do sports, the GPS module 13 detects the spot in which the person is. This spot is then identified in the local data base of the device. The user then selects the spot in which a sports session is going to be registered. A session, understood as a period of time in which the user is doing sports and tracking some relevant results, can be tracked by the device at the user's will. In other words, the user can select a menu option for starting tracking the activity registered by the device and can pause it or definitely stop it at his/her will. A session can last a whole day, or several hours or less, or be associated to the activity performed at one single spot or at different spots. The device tracks the user's activity and establishes/calculates several markers associated to the sports activity performed. Non-limiting examples of the tracked information or marker associated to the performed activity, are: starting time, ending time, duration of pauses, if any; total distance or trajectory done; amount of surfed waves (wave count) and time at which waves happen (if the user is surfing); amount of descents (descent count) and duration of the same (if the user is skiing or snow-boarding), duration of an activity, a value of velocity, a value of height, a score or a ranking.

The management and storing of the sports sessions is done locally: the information tracked is kept in the memory means (local memory) 15 until it is downloaded somewhere else, shared or deleted, at the user's will. The sessions can be synchronized with an application for a mobile phone (APP) for its enquiry at the application itself or via web (this last case only in the case in which the device has a GSM/3G module) The coordinates provided by the GPS module are used to identify, among others, the starting time, duration, tour and maximum speed reached by the user (for example on a wave during a surfing session or on a skiing or snow-boarding track during a snow session).

Next it is explained how an exemplary parameter is tracked: the detection of waves in a surfing session. The trigger for the detection of a wave is done by speed detection and by processing the signal registered by the sensor 15 comprising an accelerometer/gyroscope, as explained next. First, when the user is swimming towards a point or position in the sea at which he/she is going to wait for a wave, the accelerometer comprised in sensor 15 of the device detects the swimming stroke. At the same time, the GPS module 13, which provides exact position (coordinates: latitude, length and height) and time, is able to continuously calculate the instantaneous velocity of the user wearing/keeping the device. From the collection of coordinates, the GPS module 13 calculates the trajectory followed by the user. The detection of stroke plus the velocity and trajectory are used to establish that the user is swimming towards the location in which he will start surfing a wave. The user then stops at the place at which he/she is going to wait for a wave. The device detects the lack of swimming stroke, which means that the user is waiting for a wave to surf. Depending on the value of the instantaneous velocity, the device is able to decide whether the user is swimming before starting to surf, or not. For making such decision, a threshold is established. The threshold can be established, for example, but not limitedly, to 5 Km/h. If the velocity of the user is above 5 Km/h, the device understands that the user is either surfing or has been dragged by the wave. And the device discards that the user is swimming towards a coming wave. When it is detected that the user may be surfing a wave (because the velocity is above a established threshold), the device preferably requests information from the GPS module 13 more frequently. As can be seen, a false positive can be detected, if the velocity of the user is above the threshold because the user has been dragged by the wave/sea or the user has voluntarily left the wave. In order to discard false positives, the device analyses the duration of the user moving at said detected velocity. If the velocity of the user is above said threshold for a period of time shorter than a second threshold (a time threshold), it is decided that the user has not surfed that wave; rather, the user must have left the wave or been dragged by it. This second threshold can be alternatively established in terms of distance: if the trajectory done at a velocity above the first threshold is longer than a certain second threshold (distance threshold), it is established that the user has actually surfed a wave. A counter managed by microprocessor 11 automatically counts that one wave has been surfed, and tracks additional information, such as time, duration, trajectory and velocity. The user does not have to press any key on the device. In other words, the process is totally automatic.

The former example refers to surfing, but a similar tracking of parameters is done when the user is for example skiing: From the GPS coordinates and time, it can be established that the user is climbing in a chairlift or similar means to reach a track or the upper part of a slope. When the velocity is above a certain threshold (generally different from the threshold in a surfing session) it can be established that the user is skiing or snow-boarding. The trajectory registered by the GPS module contributes to confirm that the user is skiing.

The portable device provides a gamification option or interaction option. The portable device is designed to be used in a mobile proprietary network formed by a plurality of similar portable devices. During a sports session or activity, the RF module 14 is fixed/set to a proprietary communications protocol. This proprietary protocol permits the device to broadcast a message with information, that is to say, there is no need to establish a point-to-point connection between the device and a second device. In other words, the portable device can share the tracked markers or score on the spot, without requiring an Internet connection. This proprietary protocol works under the same physical requirements as a conventional standardized short-distance wireless communication protocol (such as Bluetooth) (frequency band of 2.4 GHz, power...). The main difference with respect to the "standardized mode" is that the conventional standardized mode (such as Bluetooth Low Power mode) requires the establishment of a point-to-point communication between two devices (one working as master and the second one working as slave). In other words, in the standardized Bluetooth Low Power mode several signaling messages are sent prior to establishing the proper exchange of user data. On the contrary, the proprietary mode (or broadcast mode) minimizes the establishment of a call, to the extent that all the messages carry user data. All the messages are broadcasted. There is no need to acknowledge the presence of other users prior to sending the messages. No acknowledgement of reception of messages is expected nor provided.

In particular, the RF module 14 broadcasts any information being tracked/calculated by the device, in relation to the sports session (score, ranking, number of surfed waves and time at which waves happen, number of ski descents and duration of the same, starting time, ending time, duration of pauses, if any; total distance or trajectory done; duration of an activity, a value of velocity, a value of height, ...). The broadcasted message comprises an identifier of the device which originally sends the information. In a particular embodiment, information is broadcasted at periodic time instants, for example every 5 seconds or every 10 seconds. Alternatively, the information is broadcasted every time a selected event happens, for example every time the device counts that a new wave has been surfed or a new ski descent has been completed. Since the RF module 14 has a typical working range of around 100-150 meters (which may vary depending on the circumstances), all surfers or skiers (or sportsmen in general) having a similar device (with that same proprietary protocol) which are within that range at the moment of broadcasting a message, will receive said message.

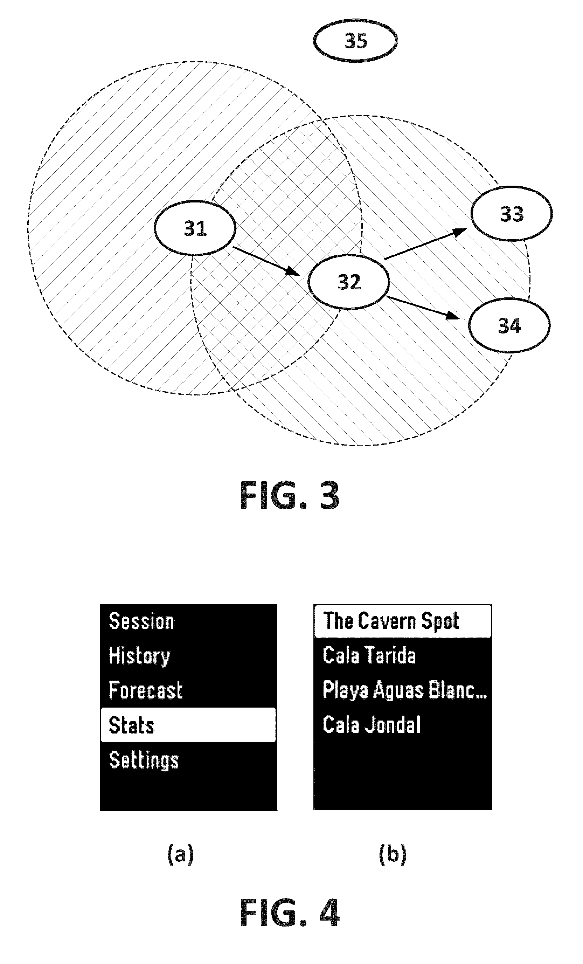

Figure 3 illustrates the gamification option done with the RF modules of the devices configured in "proprietary mode" or "broadcast mode". Five devices 31 32 33 34 35 are shown. The five devices are set to "proprietary mode" or "broadcast mode". The respective coverage or working range of devices 31 and 32 is shown (concentric circles around devices 31 32). When device 31 has a message to send (to provide the others with updated information of its performance), device 31 broadcasts a message that reaches device 32 because device 32 is within the coverage of device 31. However, device 35 does not receive the message because it is too far away. Device 32 updates its database with the received information and broadcasts the received message, which is received by devices 33 34, because both are located within the coverage of device 32. The message can be rebroadcasted by these devices 33 34, until the maximum number of hopes associated to the message are done.

In a preferred embodiment, each broadcasted message is broadcasted a certain amount of times. For example, each message is sent with a counter set at a certain value. Every time the message reaches a neighbour device for the first time, the counter is counted down, in such a way that the counter is updated by subtracting "1" to its value. When the counter reads "0", the message is not broadcasted any more. For example, a device sends a message with a counter fixed to "3". That message reaches a second device and then the counter is fixed to "2". The second device broadcasts the message again, which reaches a third device, and the counter of the message is set to "1 ". The third device broadcasts the message again, and the counter is fixed to "0", so that the message is not resent anymore. In a particular embodiment, the device originating the information broadcasts its message more than once. If a device receives a message originated by itself, the message is discarded. Every time a device receives a broadcasted message with information from another device, apart from broadcasting the message again, the device receiving the message checks if the originating device is registered as a contact and, if it is, updates its information. Each device can display the information associated to its user (summary of a session, duration, trajectories, score, ranking...) and to any other user registered as a contact. The shared information can be displayed on each device display, for example in the form of a table with the users' scores.

It might happen, especially among users who are very far away from each other, that at a certain time instant the table with users' scores that one device has is different from the table with users' scores that another device has. This is solved in several different ways explained next.

In a particular embodiment, a broadcasted message comprises, apart from an identifier of the device which originally sends the information, the whole scores table (for a group of participants) updated with the new score of the user originating that message (and the recalculation of the general score of sportsmen/women), together with the time at which it was updated for the last time. This way, it is possible to discard non-updated information and to keep the most updated version of the table. For example, when device 33 receives, probably after several hops, a broadcasted message originated in device 31, if the time associated to the received table is older than the time associated to the current table stored in that device 33, device 33 does not update its table. If, on the contrary, the time associated to the received table is more recent than the time associated to the current table stored in that device 33, device 33 updates its table.

In an alternative embodiment, a broadcasted message comprises, apart from an identifier of the device which originally sends the information, a mark or score (not a whole table), achieved by the user of the device originating that broadcasted message, and the time instant at which that mark or score was achieved. If the device that receives the message updates the score of that other person together with the time at which the last mark or score was achieved, it is possible to discard non-updated information. For example, when device 33 receives, probably after several hops, a broadcasted message originated in device 31, including a new mark registered by device 31, if the time associated to the received mark is older than the time associated to the last mark of that device 31 stored in that device 33, device 33 does not update its score associated to that other device 31. If, on the contrary, the time associated to the received mark is more recent than the time associated to the last mark of that device 31 stored in that device 33, device 33 updates its score associated to that other device 31.

In an alternative embodiment, a broadcasted message sent by device 31 comprises, apart from an identifier of the device which originally sends the information, the highest velocity reached by its user for example for the last five minutes. In this case, there is no need to send the time instant at which that mark (highest velocity) was achieved. When the device that receives the message compares the received highest velocity of device 31 to the last highest velocity of device 31 stored in its memory, it only updates its memory if the received highest velocity is higher than the already stored highest velocity for that user 31.

Another example of mark that fits in this embodiment is, for example, the longest distance performed. The RF module 14 is set to the proprietary communications mode during the sports session or activity, because it is during the session that a user is interested in sharing in real time his/her results with others and to receive others' results. When a session ends, the RF module 14 can be manually or automatically switched to conventional Bluetooth mode (master-slave mode or point-to-point mode). In a particular embodiment, when the user considers that he/she has finished his/her activity, the user manually closes the active session by selecting the corresponding option by scrolling the menu and pressing keys. When the session is closed, the mode is switched from proprietary mode to conventional master-slave mode (i.e. conventional Bluetooth). In an alternative embodiment, the closing of the session is automatic (for example if the session has been open for a time period longer than a certain established threshold, or if the resources of memory dedicated to storing the session have been used). Then, the switching from proprietary mode to conventional master-slave mode is also automatic. Once in master-slave mode, the user can then upload the registered activity tracked in a sports session (surfing, skiing or any other) somewhere else (web page, social network...), such as on a mobile phone, laptop, or the like. In the Bluetooth mode the user can also download information (such as weather forecast) from those devices. Alternatively, when a session ends, a user can keep of being set to the proprietary mode, if he/she wants to keep on receiving and sharing information from sessions of other people. The device can store in its memory several sessions (the exact amount depending on the capacity of the memory means, on the amount of information stored in each session and/or on the duration of the sessions). A session ends when the user manually stops the registration of it in the device or its duration lasts over a certain threshold.

Optionally, the device has a USB port for wired communication with other devices.

As already mentioned, the device optionally has a GSM/3G module 17 comprising conventional GSM/3G transmitting/receiving means and at least one GSM/3G antenna.

This module 17 permits real-time communication with any external source of information, such as web pages, APPs, or others. Devices having this GSM/3G module 17 can directly provide the tracked information to a web page or receive weather information (or other information) into the device.

Figure 4 shows some examples of the general menu as can be displayed on a device display. With the buttons or keys of the device, a selection can be made: Session, History, Forecast, Stats and Settings (figure 4(a)). By scrolling up/down, a selection can be made ("Stats" in figure 4(a)). Then the first row of "Stats" is highlighted ("The Cavern Spot" in figure 4(b)). Next, an exemplary implementation of the displayed options is shown in figures 5-9.

Figure 5 shows examples of the Session information as shown on a device display according to an embodiment of the invention. This embodiment refers to surfing. When "Session" is selected in the menu, the display shows that the GPS signal is being acquired (figure 5(a)). Then, several spots are displayed (figure 5(b)). When the user has selected one of them, he/she can start doing sports and the relevant information will be tracked and shown on the display (figure 5(c)). When the device detects that the user is for example surfing a wave, the screen is switched to "wave mode" and some particular real-time information is provided, such as last surfing velocity, last surfed distance and duration (figure 5(d)). That screen is displayed for a certain period, for the user to have enough time to read it. The user can close that screen at any time by pressing a button. If there are other users around with whom to compete, the user can select the "Challenge mode" (figure 5(e)) for disabling the device to "broadcast mode". The user can also close the current session or continue it. When a session ends, a screen is displayed, showing the maximum height of wave achieved during the session (figure 5(f)). When a session has ended, all the data of the complete session are shown, in "slide mode", with different information being displayed for several seconds: time, distance, maximum velocity, number of surfed waves and score of competition (figures 5(g), 5(h) and 5(i)).

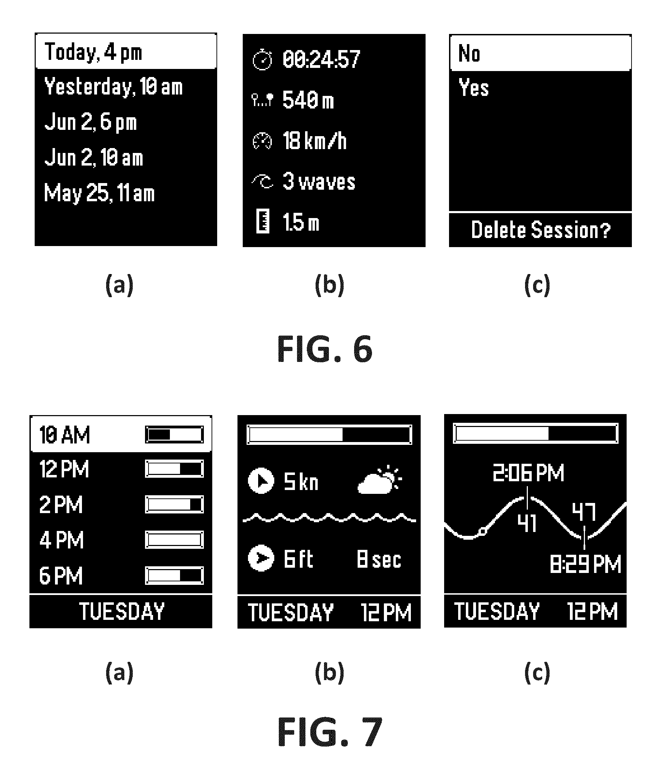

Figure 6 shows some examples of the history information as can be shown on a device display according to an embodiment of the invention. With the buttons or keys of the device, a list of stored sessions can be displayed (figure 6(a)). For each session, some information is shown (figure 6(b)), such as its duration, the total distance made, the maximum speed reached by the user and the amount of waves surfed (or descents if the user is a skier). The sessions can be deleted (figure 6(c)).

Figure 7 shows some examples of the forecast information as can be displayed on a device display device according to an embodiment of the invention. With the buttons or keys of the device, the "Forecast" menu can be displayed. A list of favorite spots is shown. The first one is the local spot. For each spot, a list of hours can be displayed, as shown in figure 7(a). For each hour, a bar indicating the quality of the spot conditions at that time is shown. After selecting one hour, detailed information for that time slot is shown (figure 7(b). By pressing the keys, the tides information is also displayed (figure 7(c)).

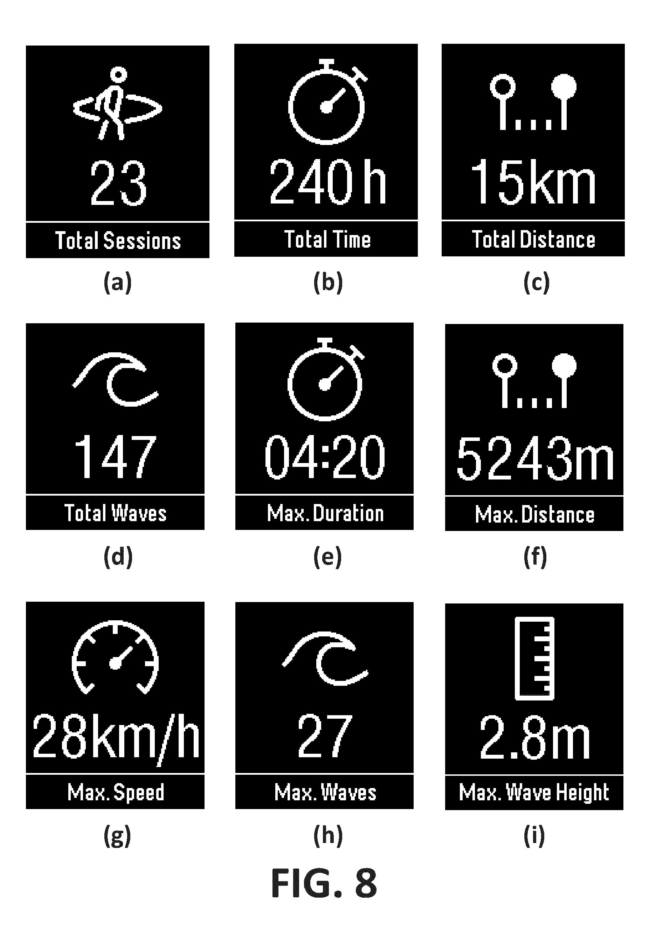

Figure 8 shows the statistics option displayed on the display of the device according to an embodiment of the invention. The statistics are displayed in "slide-mode", with different information being displayed for several seconds: number of sessions ever performed with the device (figure 8(a)), duration of all sessions ever performed (figure 8(b)), total distance ever done (figure 8(c)), total number of surfed waves (figure 8(d)), duration of longest session (figure 8(e)), longest distance done in a single session (figure 8(f)), maximum speed achieved in a session (figure 8(g)), largest amount of surfed waves in a single session (figure 8(h)) and highest wave ever surfed (figure 8(i)). The information can also be manually scrolled.

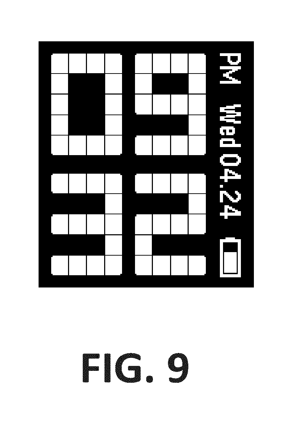

Figure 9 shows an exemplary design of the watch function (time displayed) of the device of the invention. In this exemplary implementation, the time is provided by the GPS module. Any other conventional alternative ways to provide the time are also possible.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A portable device for tracking sports activity performed by a user to whom said portable device is associated, comprising a microprocessor (11), memory means (15) for storing information, a GPS module (13) for calculating the position of the device and a display (12) for providing visual information; the portable device being configured to establish, from the information captured at least by said GPS module (13), at least one marker associated to the sports activity performed by said user and to store said at least one marker in said memory means (15);
the portable device being **characterized in that** it comprises an RF module (14) implementing a short-distance wireless protocol, said RF module (14) being configured to be set to work in broadcast mode, thus enabling the portable device to broadcast a message comprising an identifier of the portable device and said at least one marker associated to the sports activity performed by said user.

2. The device of claim 1, wherein said message comprises the maximum number of times the message is to be retransmitted.

3. The device of any preceding claims, wherein said RF module (14) is configured to be switched from broadcast mode to master-slave mode in order to either download information to the memory means (15) or to upload said at least one marker associated to the sports activity performed to another device.

4. The device of any preceding claim, further comprising at least one accelerometer (15) configured to provide additional information for the device to establish said at least one marker.

5. The device of any preceding claim, wherein said memory means (15) is configured to store at least one of the following: a plurality of sports spots, a weather forecast, tide information and information about past sports sessions.

6. The device of any preceding claim, further comprising a GSM/3G module (17).

7. The device of any preceding claim, which is implemented as a wearable device, such as a wrist watch, a bracelet or wrist device, an ankle brace or a necklace.

8. A system comprising a plurality of portable devices according to any of the preceding claims;
wherein the RF module (14) of each portable device of said plurality of portable devices is set to work in broadcast mode, in such a way that when one of said portable devices broadcasts a message comprising at least one marker associated to the sports activity performed by its user, said message can be received by the portable devices which are within the coverage area of said device broadcasting said message, in such a way that the tracked activity of the users of said devices is shared on the spot by the corresponding users.

9. A method for tracking sports activity performed by a user having an associated portable device, the method being performed in said portable device associated to a user performing said sports activity, comprising the following steps:
- calculating at least one marker associated to the sports activity performed from the information captured at least by said GPS module (13);
- storing said at least one marker in a memory means (15) of said device;
the method being **characterized in that** it further comprises:
- broadcasting a message comprising an identifier of the portable device and said at least one marker associated to the sports activity performed, said message being able to be received by any other portable device set in broadcasting mode and located within the coverage area of the portable device broadcasting said message.

10. The method of claim 9, further comprising the steps of, when a broadcasted message is received by another device:
- storing in said another device said at least one marker associated to the sports activity performed; and
- broadcasting said message from said another device;
in such a way that a plurality of devices associated to respective users can update their portable devices with the tracked activity of other devices.

11. The device of any of claims 1-7 or the method of any of claims 9-10, wherein said at least one marker associated to the sports activity performed is a number of surfed waves, a number of ski descents, a duration of an activity, a trajectory or distance of an activity, a value of velocity, a value of height, a starting time, an ending time, a duration of a pause, a score or a ranking.

12. The device of any of claims 1-7 or the method of any of claims 9-10, wherein said sports activity is surfing and said at least one marker associated to the surfing activity is the number of surfed waves.

13. The device of any of claims 1-7 or the method of any of claims 9-10, wherein said sports activity is skiing or snow-boarding and said at least one marker associated to the sports activity is the number of descents performed.

14. The device of any of claims 1-7 or the method of any of claims 9-10, wherein said message further comprises a table comprising a ranking of several user with their scores associated to a sports activity.

15. The method of either claim 9 or 10, wherein said at least one marker associated to the sports activity performed is a number of surfed waves, said step of calculating said at least one marker associated to the sports activity performed being done as follows:
- obtaining by said GPS module (13) the position of the device and time;
- calculating the instant velocity and distance performed by the user from different obtained values of position of the device and time;
- comparing said instant velocity with a velocity threshold and, if said instant velocity is above said threshold, establishing that the user of the device is either surfing or is being dragged by the wave;
- measuring the amount of time the instant velocity is above said threshold or the distance done at a velocity higher than said velocity threshold and, if said amount of time is larger than a time threshold or said distance done is larger than a distance threshold, establishing that the user has surfed a wave.
